# EUROPEAN PATENT SPECIFICATION

(11) **EP 2 282 763 B1**
(45) Date of publication and mention of the grant of the patent: **11.12.2013**
(21) Application number: 08873840.6
(22) Date of filing: 13.10.2008
(51) Int. Cl.: A61K 38/23

(54) **Process for preparing supramolecular calcitonin assemblies (SCA)**
Verfahren zur Herstellung supramolelularer Calcitoninaggregate
Procédé de préparation d'ensembles supramoléculaires de la calcitonine

(30) Priority: 07.04.2008 IN DE09142008
(43) Date of publication of application: 16.02.2011
(73) Proprietor: National Institute Of Immunology, New Delhi 110067 (IN); Indian Institute of Science, Karnataka 560 012 (IN)
(72) Inventor: SUROLIA, Avadhesha, New Delhi 110067 (IN); GUPTA, Sarika, New Delhi 110067 (IN); SINGH, Mahendra, Pal, New Delhi 110067 (IN); CHATTOPADHYAY, Tandrika, New Delhi 110067 (IN)
(74) Representative: Neuefeind, Regina
(86) International application number: PCT/IN2008/000664
(87) International publication number: WO 2009/125423

(56) References cited:
- EP-A2- 0 510 913
- WO-A-2007/067597
- WO-A2-2007/047834
- US-A1- 2002 099 013
- US-A1- 2002 132 760
- US-A1- 2004 138 095
- US-A1- 2006 014 670
- US-A1- 2007 021 345
- US-A1- 2007 178 136
- US-B1- 6 245 359
- US-B1- 6 531 448
- US-B1- 6 630 171
- AKIYOSHI K ET AL: "STABILIZATION OF INSULIN UPON SUPRAMOLECULAR COMPLEXATION WITH HYDROPHOBIZED POLYSACCHARIDE NANOPARTICLE" CHEMISTRY LETTERS, CHEMICAL SOCIETY OF JAPAN, TOKYO, JP, 1 January 1995 (1995-01-01), page 707/708, XP001027383 ISSN: 0366-7022
- BAUER H H; AEBI U; HAENER M; HERMANN R; MUELLER M;ARVINTE T; MERKLE H P: "Architecture and polymorphism of fibrillar supramolecular assemblies produced by in vitro aggregation of human calcitonin" JOURNAL OF STRUCTURAL BIOLOGY, vol. 115, no. 1, 1995, pages 1-15, XP002561190 ISSN: 1047-8477
- SHEFFIELD C A ET AL: "Safety and efficacy of exenatide in combination with insulin in patients with type 2 diabetes mellitus." ENDOCRINE PRACTICE : OFFICIAL JOURNAL OF THE AMERICAN COLLEGE OF ENDOCRINOLOGY AND THE AMERICAN ASSOCIATION OF CLINICAL ENDOCRINOLOGISTS APR 2008, vol. 14, no. 3, 1 April 2008 (2008-04-01), pages 285-292, XP9127404 ISSN: 1934-2403
- MINGDONG DONG ET AL: "AFM study of glucagon fibrillation via oligomeric structures resulting in interwoven fibrils" NANOTECHNOLOGY, IOP, BRISTOL, GB, vol. 17, no. 16, 28 August 2006 (2006-08-28), pages 4003-4009, XP020103982 ISSN: 0957-4484

## Description

### TECHNICAL FIELD

The present invention relates to protein therapeutics for treatment of chronic diseases.

### BACKGROUND OF INVENTION

Protein medications are the most rapidly expanding class of therapeutics, serving patients with diabetes, cancer, cardiovascular, renal, gastrointestinal, rheumatologic and neurological diseases among many others. The therapeutic and commercial value of protein as therapeutics including insulin, erythropoietin, G-CSF, plasminogen activator, and interferons is undisputed. Improved proteins or their formulations have enhanced the therapeutic efficacy of these parent products, by increasing their potency, time of action, and other properties.

EP 0 510 913 discloses a fibrillation process for calcitonin wherein a calcitonin powder is mixed with water or an aqueous solution and is then stirred at a temperature of 2°C to 50°C. For a solution of 200 mg/ml calcitonin in water an incubation period of one hour is needed.

Bauer et al. (1995) J. Struct. Biol. 115(1): 1-15 discloses a process for aggregating calcitonin comprising dissolving lyophilized calcitonin in ultrapure water, filtering the solution through a low protein binding sterile filter and incubating the solution at 4°C for a period ranging from hours to months.

### SUMMARY OF THE INVENTION

The present invention discloses protein therapeutics for treatment of chronic diseases. The present invention particularly discloses supramolecular protein assembly, wherein the supramolecular protein assembly comprises the insoluble and aggregated oligomeric form of the protein. The present invention also discloses the pharmaceutical composition comprising supramolecular protein assembly.

The supramolecular protein assembly and compositions disclosed in the present invention are useful for the treatment of metabolic disorders and other chronic and inflammatory diseases such as rheumatoid arthritis, osteoporosis, chronic inflammatory and peripheral pain, sepsis, and allergy where a continuous and prolonged therapy is required using peptides, proteins or small drug molecules.

One aspect of the present disclosure provides a supramolecular protein or peptide assembly (SPA) useful as protein therapeutics for the treatment of chronic diseases selected from the group consisting of osteoporosis, arthritis, cancer, endotoxic shock, wherein the assembly comprises insoluble and aggregated oligomeric form of the protein or peptide.

Another aspect of the present invention provides a supramolecular calcitonin assembly (SCA) useful as protein therapeutics for the treatment of osteoporosis, wherein the assembly comprises insoluble and aggregated oligomeric form of calcitonin.

Further the present disclosure provides a pharmaceutical composition useful as protein therapeutics for the treatment of chronic diseases selected from a group consisting of osteoporosis, arthritis, cancer, endotoxic shock, wherein the composition comprising therapeutically effective amount of the supramolecular protein or peptide (SPA) assembly disclosed in the present invention.

Also provided is a pharmaceutical composition for the treatment of osteoporosis, the composition comprising supramolecular calcitonin assembly' (SCA) of the present disclosure.

Yet another invention is to provide a process of preparation of supramolecular calcitonin assembly (SCA) disclosed in the present invention, the process comprising dissolving calcitonin at a temperature of about 25 to 60°C in a solution having pH in the range of about 4.0 to 8.0; and incubating the above for a period of 6-48 hours with constant stirring to obtain Supramolecular Calcitonin Assembly (SCA), wherein SCA comprises insoluble and aggregated oligomeric form of calcitonin

Further, the present disclosure provides a method for treating of chronic diseases selected from a group consisting of osteoporosis, arthritis, cancer, endotoxic shock, wherein the method comprising administering to a subject in need thereof a therapeutically effective amount of the pharmaceutical composition which is effective for the alleviation of the disorder or disease.

One of the aspect of the present disclosure is to provide a method for treating osteoporosis, wherein the method comprising administering to a subject in need thereof of a therapeutically effective amount of the pharmaceutical composition comprising the supramolecular calcitonin assembly for treating osteoporosis.

Yet another aspect of the present disclosure provides use of a supramolecular protein assembly for the treatment of chronic diseases selected from a group consisting of osteoporosis, arthritis, cancer and endotoxic shock.

### BRIEF DESCRIPTION OF THE DRAWINGS

Figure 1 shows kinetics of calcitonin fibril formation in PBS at 37°C under stagnant condition. Fibril formation was monitored by turbidity measurement at 400 nm.
Figure 2 shows *in vitro* release profile of calcitonin released from SCA-II under constant volume of 1 ml and dialyzing against membrane in 2% mannitol (5 ml). The release was observed by monitoring absorbance at 275 nm.
Figure 3 shows profile of calcitonin released from SCA-II in the serum of ovariectomized rat of different treatment group. Calcitonin in serum was measured by an immunoassay kit.

### DETAILED DESCRIPTION OF THE INVENTION

The present disclosure provides protein therapeutics for treatment of chronic diseases/disorders. The present disclosure particularly provides supramolecular protein or peptide (SPA) assembly, vvherein the supramolecular protein or peptide assembly (SPA) comprises the insoluble and aggregated oligomeric form of the protein, wherein the protein is calcitonin. The present disclosure also provides the pharmaceutical compositions comprising supramolecular protein assembly.

The supramolecular protein or peptide (SPA) assembly and pharmaceutical compositions disclosed herein are useful for the treatment of chronic and inflammatory diseases such as rheumatoid arthritis, osteoporosis, chronic inflammatory and peripheral pain, sepsis, and allergy where a continuous and prolonged therapy is required using peptides, proteins or small drug molecules.

The methodology of the present disclosure can also be extended for the treatment of osteoporosis using calcitonin, a peptide hormone for therapy.

The term "supramolecular protein assembly" also refers to an intermediate form of a protein formed during the process of fibrilization and is characterized by an oligomeric association of peptide/protein monomers.

The term "supramolecular calcitonin assembly" or "SCA" used herein refers to supramolecular calcitonin assembly, which is the insoluble and aggregated oligomeric form of calcitonin.

In accordance with the prevent disclosure, one embodiment provides a supramolecular protein or peptide assembly (SPA) usefid as protein therapeutics for the treatment of chronic diseases selected from the group consisting of osteoporosis, arthritis, cancer, endotoxic shock, wherein the assembly comprises insoluble and aggregated oligomeric form of said protein or peptide.

Another embodiment of the present disclosure provides the protein or a peptide for preparation of supramolecular protein assembly disclosed in the present invention, wherein the protein is calcitonin.

The present disclosure also provides a supramolecular calcitonin assembly (SCA) useful as protein therapeutics for the treatment of osteoporosis, wherein said assembly comprises insoluble and aggregated oligomeric form of calcitonin.

The supramolecular calcitonin assembly (SCA) disclosed herein upon administration releases calcitonin for about 55-60 days in a subject in need thereof.

Further the present disclosure provides the supramolecular protein assembly, wherein the peptide in assembly is coupled with a small molecule drug.

One embodiment of the present disclosure provides the supramolecular protein assembly disclosed in the present invention acts as a prodrug,

Another embodiment of the present disclosure provides the supramolecular protein assembly disclosed herein acts as a prodrug, wherein the assembly is supramolecularcalcitonin assembly (SCA).

In one embodiment of the present disclosure, there is provided the supramolecular protein assembly which is non cytotoxic.

In another embodiment of the present disclosure, there is provided the supramolecular protein assembly which is non cytotoxic, wherein the assembly is supramolecular calcitonin assembly (SCA).

In accordance with the present disclosure, one embodiment provides a pharmaceutical composition useful as protein therapeutics for the treatment of chronic diseases selected from a group consisting of osteoporosis, arthritis, cancer, endotoxic shock, wherein the composition comprising therapeutically effective amount of the supramolecular protein or peptide (SPA) assembly as disclosed herein.

One embodiment relates to the pharmaceutical composition comprising the supramolecular protein or peptide (SPA) assembly of the present disclosure, wherein the protein or a peptide is calcitonin.

In another embodiment, there is provided a pharmaceutical composition for the treatment of osteoporosis, the composition comprising supramolecular calcitonin assembly (SCA) as disclosed herein.

In another embodiment, the present disclosure provides the pharmaceutical composition comprising supramolecular calcitonin assembly (SCA) as disclosed in the present invention, wherein said composition releases calcitonin.

In another embodiment, the present disclosure provides the pharmaceutical composition comprising supramolecular calcitonin assembly (SCA) as disclosed herein, wherein said composition upon administration releases calcitonin for about 55 to 60 days.

In yet another embodiment, the present disclosure provides the pharmaceutical composition comprising supramolecular calcitonin assembly (SCA) as disclosed herein, wherein single dose of the composition upon administration releases calcitonin for about 55 to 60 days, wherein concentration of calcitonin released from supramolecular calcitonin assembly (SCA) is in the range of 15-20 pg/ml.

The pharmaceutical composition disclosed herein comprises pharmaceutically acceptable carriers, additives or diluents.

The pharmaceutical composition disclosed herein is administered intravenously, intramuscularly, orally or subcutaneously.

The pharmaceutical composition disclosed herein is administered through a device capable of releasing the composition, wherein the device is selected from a group consisting of pumps, catheters and implants.

The pharmaceutical composition disclosed herein, wherein single dose of the composition upon administration releases said protein for prolonged period.

In yet another embodiment of the present invention, there is provided a process of preparation of supramolecular calcitonin assembly (SCA) as disclosed in the present invention, the process comprises dissolving calcitonin at a temperature of about 25 to 60°C in a solution having pH in the range of about 4.0 to 8.0; and incubating the above for a period of 6-48 hours with constant stirring to obtain Supramolecular Calcitonin Assembly (SCA), wherein SCA comprises insoluble and aggregated oligomeric form of calcitonin.

In a further embodiment, process of preparation of supramolecular calcitonin assembly (SCA) as disclosed in the present invention further comprises washing the SCA with PBS; and re-suspending the SCA in water or 2% mannitol.

One embodiment provides the solution for dissolving the protein, wherein the solution is selected from a group consisting of hydrochloric or acetic acid in water having pH in the range of 1.5 to 2.5; sodium acetate buffer having pH in the range of 3.5 to 5.5; phosphate buffer (PBS) having pH 6, and citrate buffer having pH in the range of 4 to 6.

The process of preparation of supramolecular protein assembly disclosed herein comprises dissolving the protein at 37°C temperature.

The process of preparation of supramolecular protein assembly disclosed herein comprises dissolving the protein in a solution having pH 7.2.

The process of preparation of supramolecular calcitonin assembly disclosed in the present invention comprises incubation of the calcitonin in the solution for 8 hours.

The present invention further provides a method for treating chronic diseases selected from a group consisting of osteoporosis, arthritis, cancer, endotoxic shock, wherein the method comprises administering to a subject in need thereof a therapeutically effective amount of the pharmaceutical composition disclosed herein, which is effective for the alleviation of the disorder or disease.

In another embodiment of the present disclosure there is provided a method for treating osteoporosis, wherein the method comprises administering to a subject in need thereof a therapeutically effective amount of the pharmaceutical composition comprising the supramolecular calcitonin assembly disclosed herein, which is effective for the alleviation of osteoporosis.

The method for treatment using the pharmaceutical composition as disclosed herein, wherein the composition is administered intramuscularly, intra peritonealy or subcutaneously.

The present disclosure also provides use of supramolecular calcitonin assembly disclosed herein for the treatment of osteoporosis.

In still another embodiment, the present disclosure provides a composition comprising of the supramolocular protein assembly which is stable, protease resistant and has longer shelf life.

In still another embodiment, the present disclosure provides a composition comprising of the Supramolecular peptide tagged assembly which is stable, protease resistance and has longer shelf life.

In still another embodiment, the present disclosure provides a composition comprising of the supramolecular protein assembly which is stable, protease resistance and has longer shelf life ranging from about 10 days to 150 days or more.

Also within the scope of the present disclosure is a variety of supramolecular protein or peptide assembly (SPA), wherein the SPA comprises the insoluble and aggregated oligomeric form of the protein or peptide, wherein the protein or peptide is useful as therapeutic protein.

As will be appreciated by those in the art a variety of the solutions such as known buffers can be used for re-suspension and washing of the supramolecular protein assembly disclosed herein.

The term "therapeutically effective amount" as used herein refers to an amount of a therapeutic agent to treat, ameliorate, or prevent a desired disease or condition, or to exhibit a detectable therapeutic or preventative effect. The precise effective amount for a subject will depend upon the subject's size and health, the nature and extent of the condition, and the therapeutics or combination of therapeutics selected for administration. The effective amount for a given situation is determined by routine experimentation and is within the judgment of the clinician.

For purposes of the present disclosure, effective dose of SCA will generally be from about 0.1 mg/kg to about 0.3 mg/kg, or about 0.3 mg/kg to about 0.8 mg/kg or about 0.5 mg/kg to about 1.0 mg/kg of the compositions of the present disclosure in the subject to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles. Typically, the therapeutic compositions are prepared as injectables, either as liquid solutions or suspensions; solid forms suitable for solution in, or suspension in, liquid vehicles prior to injection can also be prepared. Liposomes and neosomes are included within the definition of a pharmaceutically acceptable carrier. Pharmaceutically acceptable salts can also be present in the pharmaceutical composition, e.g., mineral acid salts such as hydrochlorides, hydrobromides, phosphates, sulfates, and the like; and the salts of organic acids such as acetates, propionates, malonates, benzoates, and the like.

The pharmaceutical compositions can be prepared in various forms, such as granules, tablets, pills, suppositories, capsules, suspensions, salves, lotions and the like. Pharmaceutical grade organic or inorganic carriers and/or diluents suitable for oral and topical use can be used to make up compositions containing the therapeutically-active compounds. Diluents known to the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents.

The pharmaceutical compositions disclosed herein are useful for the treatment of chronic and inflammatory diseases such as rheumatoid arthritis, osteoporosis, chronic inflammatory and peripheral pain, sepsis, and allergy where a continuous and prolonged therapy is required using peptides, proteins or small drug molecules.

The compositions disclosed herein comprise supramolecular protein assemblies of the relevant/applicable therapeutic proteins and are applicable for treatment of a number of chronic diseases and acute symptoms.

The compositions disclosed herein comprises oligomers of therapeutic proteins particularly the supramolecular assembly of a protein for sustained release of the protein.

Some widely-used biopharmaceuticals such as insulin, glucagon, and calcitonin can be induced to form amyloids. Compared to soluble precursor proteins, amorphous aggregates formed as a prelude to amyloid formation, gain new properties such as enhanced stability, protease resistance, self-propagation, longer shelf life, highly organized structure and can serve as a concentrated compact source of pure molecules.

The supramolecular protein assemblies disclosed herein exist with in a defined structure having both α-helical and β-sheet components.

One embodiment of the present disclosure provides a process for preparation of supramolecular oligomers of protein for the treatment of various diseases. The inventors also disclose acute and chronic diseases such as osteoporosis, inflammatory and chronic pain, rheumatoid arthritis, arthritic pain, cancer, endotoxic shock, etc where a similar approach to transform the peptide or present the drug molecule attached to such a transformed 'supramolecularly oligomerized proteins or peptides' and use them as a depot of the native drug has given good results.

In yet another embodiment, the current methodology can be extended to those chronic and inflammatory diseases where a sustained and continuous therapy is required using peptides, proteins or small molecules.

Another embodiment of the present invention relates to the formation of supramolecular assembly of calcitonin and its therapeutic utilization for the treatment of various diseases.

In still another embodiment of the present disclosure, supramolecular oligomer of salmon calcitonin was used for the treatment of osteoporosis in diseased subjects.

According to one embodiment of the present invention, the formation of amyloid fibrils by salmon clacitonin is concentration dependent and is completely formed by 44 h.

In yet another embodiment, the supramolecular calcitonin assembly (SCA) I, II and III are formed by 6, 8 and 12 h respectively.

Further, in another embodiment of the present invention, the release of free calcitonin from SCA follows a bell shaped curve (in small volume), with continuous release through a dialysis membrane (in large volume of aqueous solvent).

In still another embodiment of the present invention, the release of calcitonin monomers from fully grown fibrils was negligible.

In yet another embodiment of the present invention, the supramolecular calcitonin assembly (SCA) was incorporated into Alzet pumps, from which a slow and controlled release of calcitonin monomers at a rate of 2.5 µl/h is obtained.

According to one embodiment of the present invention, ovariectomised rats were administered vehicle or human/salmon calcitonin either intermittently by subcutaneous injection or continuously by Alzet osmotic minipumps containing 200 µl of either Calcitonin (CT) or supramolecular calcitonin assembly (SCA, 2mg/ml) in 2% mannitol.

In still another embodiment, a dosage of 8 U/kg b wt was given to ovariectomised rats through the pump and 16 U / kg b wt subcutaneously on alternate days.

In yet another embodiment, demeclocycline and calcein was administered 10 and 3 days before sacrifice, respectively, at a dosage of 15 mg/kg b wt.

According to one embodiment of the present invention, the serum levels of calcitonin was maintained at a higher level (19pg/ml) in rats treated with SCA compared to that of other groups.

In still another embodiment of the present invention, the serum level of calcitonin was maintained at a higher level for upto 60 days with a single pump containing the SCA.

In yet another embodiment of the present invention, supramolecular calcitonin treated rats' demonstrated marginal increase in their body weight, calcium and phosphorous levels, in comparison to ovariectomised rats given vehicle which had an increase in their body weight by almost 11% during the experimental time frame and an increase in calcium and phosphorous levels in serum.

Further, in another embodiment of the present invention, negligible levels of antibodies were found against salmon calcitonin in the serum of the treated animals.

The present disclosure provides the usage of supramolecular assembly of calcitonin as a potential therapeutic candidate for the treatment of osteoporosis in ovariectomised rats. Details are provided in Example 1. Supramolecular calcitonin assembly or SCA is incorporated into Alzet pumps, from where there is a slow and continuous release of calcitonin into the blood. This slow release of calcitonin alleviates the symptoms of osteoporosis, maintaining normal body weight and serum calcium and phosphorous levels.

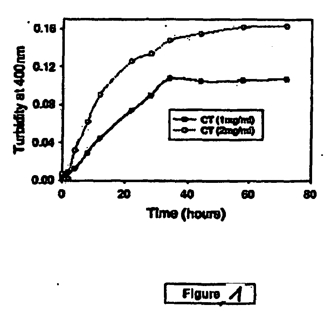

A solution of Salmon calcitonin- 1 and 2 mg/ml was prepared in PBS and incubated at 37°C for 80 hrs. The kinetics of calcitonin amyloid fibril formation was monitored by measuring turbidity at 400nm at different time interval. As shown in Fig 1 the rate of fibril formation is concentration dependent and almost completed after 44 h. The SCA-I, SCA-II and SCA-III were formed at 6, 8 and 12 h, respectively.

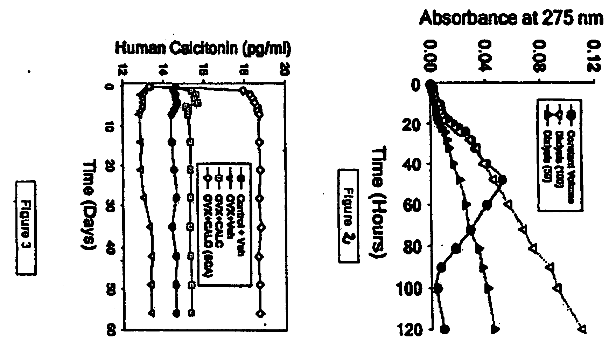

The release of free calcitonin from the SCA was monitored in 2% mannitol in a constant volume of 1 ml and by dialyzing through a dialysis membrane. The release was monitored by absorbance at 275 nm. As shown in Fig 2, the release of calcitonin from SCA in a constant volume of 1 ml follows a bell shaped curve. However, continuous release was observed under dialysis through a membrane. The release of calcitonin from fully grown calcitonin fibers was very slow and could be considered negligible for further experiments. The calcitonin SCA was further used for the *in vivo* experiments for the treatment of osteoporosis in ovariectomized rat.

As mentioned in experimental section, five groups of rats (10 rats in each group) were given different treatment. The body weight and serum profile of calcium, phosphorous and calcitonin was monitored. The release kinetics of salmon calcitonin from SCA in ovariectomized rats treated with SCA in Alzet and endogenous rat calcitonin in control rats was determined and is shown in fig 3. Calcitonin level was maintained at a relatively higher level in rats treated with SCA, compared to that of other groups. This shows that the calcitonin SCA solution in Alzet pump releases calcitonin in the biologically active form with a constant and slow rate, which in turn treated the symptoms of osteoporosis in OVX rat for upto 60 days compared to daily treatment with soluble calcitonin.

The final body weight for the four groups of rats was monitored. Rats from all four groups gained weight during the course of the study. The vehicle-treated OVX rats weighed approximately 11% more than vehicle-treated control rats at the end of the 8 week study (328 ± 19g vs. 296 ± 09g, p < 0.05). The mean body weight of OVX rats treated with CT intermittently or continuously with SCA were significantly decreased compared with that of vehicle-treated OVX rats.

Serum biochemical data are listed in Table 1. The serum CT concentration was measured in all groups during and at the end of 8 week in OVX rats treated with SCA and was significantly higher than that of vehicle-treated OVX, but not than that of control rats. Mean serum calcium levels in OVX rats treated with SCA was significantly lower than that of all other groups. Mean serum phosphorus level was also significantly increased in vehicle-treated OVX rats relative to vehicle-treated control rats. In contrast, treatment of OVX rats with CT either intermittently or continuously in case of SCA, significantly decreased serum phosphorus concentration compared to that of vehicle treatment to OVX rats. Furthermore, this variability in OVX rats treated with SCA was significantly lower than that in OVX rats treated with CT intermittently.

Screening the antibody against salmon calcitonin in all the groups showed negligible antibody in case of control, vehicle treated and calcitonin SCA treated rats. However, lower level of antibody raised against salmon calcitonin in calcitonin treated group (intermittently or continuously).

Thus the present disclosure can be further extended to all those diseases such as chronic pain, sepsis, arthritis, osteoporosis, inflammation, etc, where a continuous infusion of the therapeutic drug is required, be it a peptide, protein or a small drug molecule. This methodology of utilizing the oligomers of the drug as a depot for treatment can be extended to many more diseases, having a all round, broad spectrum applicability.

The following examples are given by the way of illustration of the invention contained in the present invention and therefore should not be construed to limit the scope of the present invention.

### EXAMPLES

It should be understood that the following examples described herein are for illustrative purposes only and that various modifications or changes in light of the specification will be suggestive to person skilled in the art and are to be included within the spirit and purview of this application and the scope of the appended claims.

### Example 1

### Calcitonin fibril formation

1 and 2 mg/ml of salmon calcitonin was prepared in PBS and incubated at 37°C for 80hrs. The kinetics of calcitonin amyloid fibril formation was monitored by measuring turbidity at 400nm at different time interval. As shown in Fig 1 the rate of fibril formation is concentration dependent and almost completed after 44 h. The SCA-I, SCA-II and SCA-III were formed at 12, 22 and 28h, respectively.

### In vitro release of calcitonin from Supramolecular calcitonin assembly

The release of free calcitonin from the SCA was monitored in 2% mannitol in constant volume of 1 ml and by dialyzing trough dialysis membrane. The release was monitored by absorbance at 275 nm. As shown in Fig 2 the release of calcitonin from SCA in constant 1 ml follows bell shaped curve. However, continuous release was observed under dialysis through membrane. The release of calcitonin from fully grown calcitonin fiber was very slow and could be considered negligible for further experiment. The calcitonin SCA was further used for the *in vivo* experiments for the treatment of osteoporosis in ovariectomized rat.

### Model for osteoporosis and treatment using calcitonin

44 female Sprague-Dawley rats (Charles River Laboratory, Wilmington, MA), approximately 90 days of age with an average body weight of 230 g were grouped into four groups at the beginning of the study. All rats were anesthetized with an intraperitoneal injection of ketamine hydrochloride and xylazine at doses of 50 and 10 mg/kg body weight (BW), respectively. Bilateral ovariectomies were performed in three groups from a dorsal approach. All rats were housed individually at 25°C with a 12 h light/12 h dark cycle. The food consumption of ovariectomized (OVX) rats was restricted to that of the control rats to minimize increase in the body weight associated with ovariectomy. All rats were treated for 8 weeks with vehicle or Human/salmon CT (Sigma) either intermittently by subcutaneous injection or continuously by Alzet osmotic minipumps (Model 2ML4; Alza Corp., Palo Alto, CA) containing 200 µl of either CT (4 µg/kg) or SCA (Supramolecular calcitonin assembly) (2 mg/ml) in 2% mannitol, designed to deliver the solutions at a constant rate of 2.5 µl/h for 28 days. The intermittent treatments started a day after surgery. The minipumps for continuous CT or Calcitonin SCA or vehicle treatments were implanted at the time of surgery (day 0) and replaced after 3 weeks in case of continuous CT and vehicle treatment. However SCA injected during this period formed a depot which keeps on releasing calcitonin for upto about 6-8 weeks. For subcutaneous injections of CT, the hormone was dissolved in a vehicle of 2% mannitol. A dose of 16 IU/kg (4µg/kg) body weight was chosen based on positive results from previous studies in OVX rats.

Half of the OVX rats from this group were injected subcutaneously on alternate days with 2% mannitol as a vehicle. The remaining OVX rats in this group were implanted subcutaneously with Alzet osmotic minipumps for continuous infusion of vehicle. Because statistically significant differences in bone variables were not observed between OVX rats treated with vehicle intermittently or continuously, the data from these subgroups have been combined.

Each OVX rat of one group was injected subcutaneously on alternate days with salmon CT at a dose of 16 U/kg BW (4 µg/kg b wt), and the other was implanted subcutaneously with an Alzet osmotic minipump for continuous infusion of human CT. The daily dose delivered to each rat by the minipump was 8 U/kg BW (2 µg/kg BW), which was equivalent to the dose given to the preceding group through subcutaneous injections on alternate days (16 U/kg).

Serum samples were stored at -80°C until analyzed for their CT concentration by immunoassay methods with a commercially available kit (Bio-Merica). This kit was used to successfully measure serum salmon CT in rats in one of our previous studies. 15 Serum samples were also analyzed spectrophotometrically for their calcium and phosphorus contents by the cresolphthalein complexone and ammonium molybdate colorimetric methods, respectively (Table 1)**.** Figure 3 shows profile of calcitonin released from SCA-II in the serum of ovariectomized rat of different treatment group. Calcitonin in serum was measured by an immunoassay kit.

The antibody generated against salmon calcitonin in all the treated groups was monitored by indirect ELISA using monoclonal antibody for calcitonin. Data are expressed as the mean ± standard deviation (SD) for each group. Statistical differences were evaluated by analysis of variance. P values < 0.05 were considered to be significant.

**Table 1: Body weight and serum calcitonin, calcium, and phosphorus**

| **Groups** | **Body Wt.** | **Calcitonin** | **Calcium** | **Phosphorus** |
|---|---|---|---|---|
| Cont + VEH | 296 ± 09 | 14.6 ± 2.9 | 10.0 ± 0.1 | 6.7 ± 0.6 |
| OVX + VEH | 328 ± 19 | 13.3 ± 2.1 | 11.2 ± 0.4 | 8.5 ± 1.1 |
| OVX+CT | 274 ± 10 | 15.3 ± 4.6 | 10.3 ± 0.4 | 7.6 ± 0.8 |
| OVX+CT(MP) | 280 ± 11 | 18.7 ± 5.0 | 9.5 ± 0.4 | 6.1 ± 0.7 |
| OVX+ SCA (MP) | 276 ± 0.9 | 19.1 ± 4.2 | 9.3 ± 0.3 | 6.0 ± 0.6 |

| | | | | |
|---|---|---|---|---|
| Data are expressed as mean ± SD. p < 0.05 OVX +SCA vs. OVX + VEH; | | | | |

## Claims

1. A process of preparation of supramolecular calcitonin assembly (SCA) wherein said assembly comprises insoluble and aggregated oligomeric form of calcitonin, said process comprising;
a. dissolving calcitonin at a temperature of about 25 to 60°C, preferably 37°C, in a solution having pH in the range of 4.0 to 8.0; and
b. incubating the above for a period of 6 to 48 hours with constant stirring to obtain Supramolecular Calcitonin Assembly (SCA), wherein SCA comprises insoluble and aggregated oligomeric form of calcitonin.

2. The process as claimed in claim 1, wherein said process further comprises
c. washing said SCA with PBS; and
d. re-suspending said SCA in water or 2% mannitol.

## Patentansprüche

1. Verfahren zur Herstellung einer supramolekularen Calcitonin-Ansammlung (SCA), wobei die Ansammlung unlösliche und aggregierte oligomere Formen von Calcitonin umfasst, wobei das Verfahren umfasst:
a. Auflösen des Calcitonins bei einer Temperatur von etwa 25 bis 60°C, bevorzugt 37°C, in einer Lösung, die einen pH im Bereich von 4,0 bis 8,0 aufweist; und
b. Inkubieren der Lösung für einen Zeitraum von 6 bis 48 Stunden unter konstantem Rühren, um die supramolekulare Calcitonin-Ansammlung (SCA) zu erhalten, wobei SCA unlösliche und aggregierte oligomere Formen von Calcitonin umfasst.

2. Verfahren wie in Anspruch 1 beansprucht, wobei das Verfahren weiter umfasst:
c. Waschen des SCA mit PBS; und
d. Resuspendieren des SCA in Wasser oder 2% Mannitol.

## Revendications

1. Procédé d'élaboration d'assemblage supramoléculaire de calcitonine (SCA), dans lequel ledit assemblage comprend une forme oligomérique insoluble et agrégée de calcitonine, ledit procédé comprenant :
a. la dissolution de calcitonine à une température d'environ 25 à 60°C, de préférence 37°C, dans une solution ayant un pH compris dans la plage de 4,0 à 8,0 ; et
b. l'incubation de la susmentionnée pendant une période de 6 à 48 heures avec brassage constant pour obtenir un Assemblage Supramoléculaire de Calcitonine (SCA), où le SCA comprend une forme oligomérique insoluble et agrégée de calcitonine.

2. Le procédé tel que revendiqué dans la revendication 1, dans lequel ledit procédé comprend en outre
c. le lavage dudit SCA avec du PBS ; et
d. la remise en suspension dudit SCA dans de l'eau ou du mannitol à 2%.
